# EUROPEAN PATENT SPECIFICATION

(11) **EP 1 889 629 B1**
(45) Date of publication and mention of the grant of the patent: **27.08.2008**
(21) Application number: 06253596.8
(22) Date of filing: 10.07.2006
(51) Int. Cl.: A61K 38/55, A61K 31/395, A61K 31/54, A61K 9/20

(54) **Stable formulation comprising a combination of a moisture sensitive drug and a second drug and manufacturing procedure thereof**
Stabile Zubereitung enthaltend eine Kombination aus einer feuchtigkeitsempfindlichen aktiven Substanz und einer zweiten aktiven Substanz und Verfahren zur Herstellung der Zubereitung.
Préparation stable contenant une combinaison d'une substance active sensible à l' humidité et d'une deuxième substance active et procédure de fabrication de la préparation.

(43) Date of publication of application: 20.02.2008
(73) Proprietor: Teva Pharmaceutical Industries Ltd, 49131 Petah Tiqva (IL)
(72) Inventor: Fox, Michael, Tel-Aviv 67291 (IL)
(74) Representative: Russell, Tim

(56) References cited:
- WO-A-03/077929
- WO-A-20/04014343
- US-A- 5 006 344
- GU L ET AL: "DRUG-EXCIPIENT INCOMPATIBILITY STUDIES OF THE DIPEPTIDE ANGIOTENSIN-CONVERTING ENZYME INHIBITOR, MOEXIPRIL HYDROCHLORIDE: DRY POWDER VS WET GRANULATION" PHARMACEUTICAL RESEARCH, NEW YORK, NY, US, vol. 7, no. 4, 1990, pages 379-383, XP009009003 ISSN: 0724-8741

## Description

### FIELD OF THE INVENTION

The present invention relates to stable pharmaceutical compositions comprising a combination of a moisture sensitive active pharmaceutical ingredient (drug), in particular an angiotensin converting enzyme (ACE) inhibitor such as Cilazapril, and a second drug, such as Hydrochlorothiazide, as the active ingredients and methods for preparing such stable pharmaceutical compositions.

### BACKGROUND OF THE INVENTION

Cilazapril is apparently an angiotensin converting enzyme ("ACE") inhibitor, which enzyme inhibits the formation of angiotensin II from angiotensin I by inhibiting the angiotensin converting enzyme. Chemically, Cilazapril is reported to be (1S,9S)-9-[(S)-1-Ethoxycarbonyl-3-phenylpropylamino]-10-oxoperhydropyridazino[1,2-a][1,2]diazepine-1-carboxylic acid and is understood to be disclosed in U.S. Patent No. 4,512,924. Cilazapril has been prescribed in treating patients suffering from hypertension. Cilazapril has the following general formula:

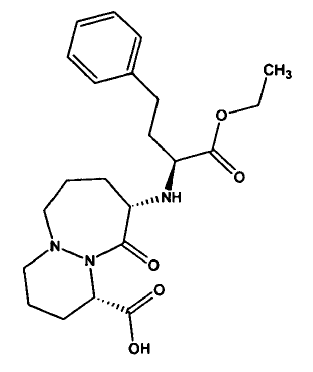

Hydrochlorothiazide is apparently a diuretic and an antihypertensive. Chemically, Hydrochlorothiazide, a 3,4-dihydro derivative of chlorothiazide, is reported to be 6-chloro-3,4-dihydro-2H-1,2,4-benzothiazidine-7-sulfonamide 1,1-dioxide and has the following general formula.

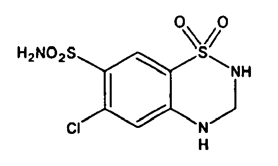

Hydrochlorothiazide is indicated as adjunctive therapy in edema associated with congestive heart failure, hepatic cirrhosis, and corticosteroid and estrogen therapy. It has also been found useful in edema due to various forms of renal dysfunction such as nephrotic syndrome, acute glomerulonephritis, and chronic renal failure. Further, Hydrochlorothiazide is indicated in the management of hypertension either as the sole therapeutic agent or to enhance the effectiveness of other antihypertensive drugs in the more severe forms of hypertension.

One of the requirements for an acceptable pharmaceutical composition is that it must be stable. A stable pharmaceutical composition does not exhibit substantial decomposition of the active pharmaceutical ingredient during the time between the manufacture of the composition and its use by a patient. Cilazapril and a number of other drugs suffer from instability problems because the active pharmaceutical ingredient rapidly degrades in the presence of water/moisture. Such active pharmaceutical ingredients (drugs) can therefore be characterized as moisture-sensitive drugs.

It is known that, tablet blends may be dry mixed, dry-granulated or wet-granulated before tableting. The choice of the processing procedure, dry mixing, dry granulation, wet granulation, or some other granulation process, depends on the properties of the drug and the chosen excipients. Generally, a dry manufacturing process is thought to be preferable for moisture-sensitive drugs.

To improve the stability of moisture sensitive drugs, water scavenger compounds may be incorporated into a tablet matrix. One such a water scavenger compound is the binder Copovidone (Plasdone S-630^{®}), which binder is specifically recommended for moisture sensitive drugs. However, with very little success attempts were made to formulate Cilazapril tablets using this material in a dry granulation process. In such Cilazapril tablets degradation of the active pharmaceutical ingredient was apparent.

Wet-granulation processes have not been considered appropriate for moisture sensitive drugs since the very nature of these processes can include the presence of water/moisture. However, as described in co-pending European patent application No. 06252877.3, filed June 2, 2006, the best stability results can be achieved with a composition or formulation comprising the moisture sensitive drug and a binder such as Copovidone, wherein the formulation/ composition is prepared using a wet granulation process, comprising wetting and then drying the composition at an elevated temperature.

Pharmaceutical compositions of such moisture sensitive active pharmaceutical ingredients (active drug substances) may contain one or more additional drug substances in a combination pharmaceutical composition. Such combination pharmaceutical compositions could provide enhanced treatment effectiveness or provide treatment while ameliorating undesired side effects of one such moisture sensitive active pharmaceutical ingredient. However, the combination of active pharmaceutical ingredients in one composition requires that the active pharmaceutical ingredients are compatible in terms of activity, side effects, and effectiveness for example.

Hydrochlorothiazide (HCTZ) can be used in combination with other antihypertensives. Moreover, pharmaceutical compositions for use in the treatment of hypertension may comprise a combination of Hydrochlorothiazide and an antihypertensive agent. Furthermore, HCTZ is compatible with the moisture sensitive active pharmaceutical ingredient Cilazapril for inclusion in a combination pharmaceutical composition. However, a pharmaceutical composition comprising a moisture sensitive drug substance and a second drug substance prepared using a single wet granulation process of the combined active ingredients appeared not to be a stable pharmaceutical composition but showed degradation of Cilazapril.

Surprisingly, the best stability results can be achieved, if a wet granulation process for preparing the combination pharmaceutical composition will be divided in at least two steps, wherein the second drug substance, preferably Hydrochlorothiazide (HCTZ), is added to the wet granulate after the wet granulation of the moisture sensitive drug substance, preferably Cilazapril, is completed.

### SUMMARY OF THE INVENTION

The present invention provides stable pharmaceutical compositions comprising a combination of active ingredients, and methods of their preparation. In a preferred embodiment, the present invention provides stable pharmaceutical compositions of a combination of Cilazapril and Hydrochlorothiazide (HCTZ), and methods of their preparation.

In one aspect the present invention provides a pharmaceutical composition comprising;
a) a moisture sensitive active pharmaceutical ingredient; and
b) a second active pharmaceutical ingredient;
wherein the moisture sensitive active pharmaceutical ingredient is first wet granulated with a solution of at least one pharmaceutical excipient in at least one processing solvent before granulation with the second active pharmaceutical ingredient. Preferably, the excipient is a binder.

In another aspect the present invention provides a method of preparing a pharmaceutical composition comprising a moisture sensitive active pharmaceutical ingredient and a second active pharmaceutical ingredient comprising the following steps of:
a) providing a moisture sensitive active pharmaceutical ingredient;
b) mixing the moisture sensitive active pharmaceutical ingredient with at least one pharmaceutically acceptable excipient, forming a mixture; and
c) wet granulating the mixture with a solution of a binder excipient dissolved in one or more processing solvents forming a wet granulate;
d) providing a material comprising a second active pharmaceutical ingredient and optionally one or more pharmaceutical excipients; and
e) adding the material from step d) to the wet granulate from step c) forming a combined granulate,
wherein when the material of step d) comprises a second pharmaceutical ingredient and one or more pharmaceutical excipients the material is optionally a mixture obtained by mixing the second pharmaceutical ingredient with the one or more pharmaceutical excipients.

In another embodiment of the present invention the method further comprises steps of preparing a tablet pharmaceutical composition of the present invention wherein the method further comprises the steps of:
f) mixing the combined granulate with one or more excipients forming a final blend;
g) pressing the final blend into a tablet; and
h) optionally coating the tablet with a cosmetic coat.

The present invention also provides a method of treating a patient suffering from a disease, preferably hypertension, comprising administering to a patient in need thereof a therapeutically effective amount of a stable pharmaceutical composition comprising a moisture sensitive active pharmaceutical ingredient, preferably Cilazapril, a second active pharmaceutical ingredient, preferably Hydrochlorothiazide, and at least one pharmaceutical excipient, wherein the active pharmaceutical ingredients are wet granulated with a solution of the at least one pharmaceutical excipient.

### DETAILED DESCRIPTION OF THE INVENTION

As used herein the term moisture sensitive active pharmaceutical ingredient refers to an active pharmaceutical ingredient which rapidly degrades in the presence of water/moisture.

In understanding the term "moisture sensitive active pharmaceutical ingredient" it is helpful to consider how the exemplified Cilazapril behaves on storage. When commercially available Cilazapril tablets (Vascace^{®}) are stored at 55°C and 75% relative humidity in the marketed package, "aluminum-aluminum cold form blisters", which are considered the "gold standard" with respect to moisture barrier blister packs, the degradation as evidenced by formation of Cilazaprilat is of the order of one percent or so over the initial Cilazaprilat content as compared with the initial Cilazapril content, over a seven day storage period, whereas when the tablets are removed from this package and stored at the same conditions and thus exposed to moisture, for just 48 hours, greater than five times that Cilazaprilat formation is observed, see Table 1 below. It is this type of characteristic that identifies Cilazapril as a moisture sensitive active pharmaceutical ingredient.

**Table 1**

| Lot No | Storage conditions | Storage period, hours | Total Degradation Products, % |
|---|---|---|---|
| # B2017 | Packed | Time "0" | 1.1 |
| # B2017 | Packed | 168 | 2.1(1%) |
| # B2017 | Packed | 336 | 2.8(1.7%) |
| # B2017 | Unpacked | 48 | 7.5(6.4%) |

Wet-granulation processes have not been considered appropriate for moisture sensitive drugs since the very nature of these processes can include the presence of water/moisture. However, as described in co-pending European patent application No. 06252877.3, filed June 2, 2006, the best stability results can be achieved with a composition or formulation comprising the moisture sensitive drug and a binder such as Copovidone, wherein the formulation/composition is prepared using a wet granulation process, comprising wetting and then drying the composition at an elevated temperature.

Pharmaceutical compositions of such moisture sensitive active pharmaceutical ingredients (active drug substances) may contain one or more additional drug substances in a combination pharmaceutical composition. Such combination pharmaceutical compositions could provide enhanced treatment effectiveness or provide treatment while ameliorating undesired side effects of one such moisture sensitive active pharmaceutical ingredient. However, the combination of active pharmaceutical ingredients in one composition requires that the drug substances are compatible in terms of activity, side effects, and effectiveness for example. A compatible second drug substance for inclusion in a combination pharmaceutical composition comprising the moisture sensitive drug substance Cilazapril, is for example Hydrochlorothiazide (HCTZ).

However, a pharmaceutical composition comprising a moisture sensitive drug substance and a second drug substance using wet granulation of the combined drug substances appeared not to be a stable pharmaceutical composition. In particular, such pharmaceutical composition containing only Cilazapril as its active pharmaceutical ingredrient was shown to be stable but showed unacceptable degradation to Cilazaprilat, when Cilazapril was combined with a second drug substance such as HCTZ in a composition where a combined mixture of the drug substances was wet granulated.

Surprisingly, the best stability results can be achieved, if a wet granulation process for preparing the combination pharmaceutical composition will be divided in at least two steps, wherein the second drug substance, preferably Hydrochlorothiazide (HCTZ), is added to the wet granulate after first the granulation of the moisture sensitive drug substance, preferably Cilazapril, is completed. HCTZ can be added to the wet granulate alone or together with other ingredients and/or in a granulation solution.

In one aspect the present invention provides a pharmaceutical composition comprising;
a) a moisture sensitive active pharmaceutical ingredient, preferably Cilazapril; and
b) a second active pharmaceutical ingredient, preferably Hydrochlorothiazide;
wherein the moisture sensitive active pharmaceutical ingredient is first wet granulated with at least one pharmaceutical excipient in at least one processing solvent before granulation with the second active pharmaceutical ingredient.

In the context of the present invention, the first wet granulation step should not involve a significant proportion of the second active pharmaceutical ingredient, and most preferably should not contain any of the second active pharmaceutical ingredient.

Preferably, the at least one excipient is a binder and the pharmaceutical composition comprises at least two pharmaceutical excipients.

Preferably the amount of the moisture sensitive active pharmaceutical ingredient in the composition is about 0.1% to about 25%, more preferably about 0.5% to about 15%, of the total weight of the composition. A most preferred amount of the moisture sensitive active pharmaceutical ingredient in the composition is about 0.6% to about 2.7% of the total weight of the composition. Preferably, the moisture sensitive active pharmaceutical ingredient is Cilazapril.

Preferably the amount of the second active pharmaceutical ingredient in the composition is about 1% to about 25%, more preferably of about 3% to about 15%, of the total weight of the composition. A most preferred amount of the second active pharmaceutical ingredient in the composition is about 5% to about 10% of the total weight of the composition. Preferably, when the moisture sensitive active ingredient is an "ACE inhibitor" the second active pharmaceutical ingredient is preferably a diuretic drug; more preferably a thiazide; most preferably Hydrochlorothiazide (HCTZ).

The present invention further provides a stable pharmaceutical composition comprising a moisture sensitive active pharmaceutical ingredient, a second active pharmaceutical ingredient and at least one pharmaceutically acceptable excipient, wherein the composition contains not more than 3% (w/w of the initial amount of the moisture sensitive active pharmaceutical ingredient) of a major degradation product after storage in a package with moisture barrier properties which properties are at least as efficient as aluminum-aluminum cold form blisters. Preferably, the concentration of the major degradation product in the stable pharmaceutical composition of the present invention after storage as described above is not more than 2%. More preferably, the concentration of the major degradation product in the stable pharmaceutical composition of the present invention after storage as described above is not more than 1%. Storage may comprise storage at a temperature of 55°C for 14 days and storage at a temperature of 40°C and 75% relative humidity for three months. The degradation product may be detected by HPLC analysis. Preferably, the moisture sensitive active pharmaceutical ingredient is Cilazapril and the degradation product is its major degradation product Cilazaprilat. Preferably, the second pharmaceutical ingredient is HCTZ. It is understood that Cilazaprilat has the following structure:

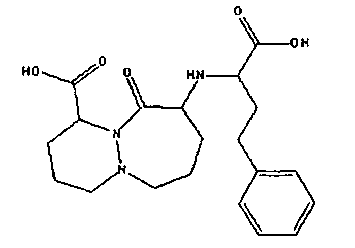

A stable pharmaceutical composition of the present invention therefore provides a pharmaceutical composition of a moisture sensitive active pharmaceutical ingredient and a second pharmaceutical ingredient, preferably Cilazapril and HCTZ respectively, characterized by comprising not more than 3%, preferably not more than 2%, most preferably not more than 1%, by weight per weight of the total amount of moisture sensitive pharmaceutical ingredient, Cilazapril, of its major degradation Cilazaprilat product upon storage.

Preferably, the stable pharmaceutical composition of the present invention comprises at least about 4% of a binder by total weight of the composition. Preferably, the pharmaceutical composition comprises from about 4% to about 20%, more preferably from about 5% to about 10% of a binder by total weight of the composition. The binder comprises for example, one or more of, a cellulose derivative, a polyvinyl pyrrolidone (PVP) and its derivatives, a polyvinylacetate (PVA) or a polyvinyl alcohol. Examples of suitable cellulose derivatives as a binder in the present invention are Hydroxypropylmethyl cellulose (HPMC) or Hydroxypropyl cellulose (HPC). More preferably, the binder is the Copovidone, exemplified by Plasdone^{®} S-630 (Copovidone), which is a synthetic, 60:40, linear, random copolymer of N-vinyl-2-pyrrolidone and vinyl acetate, and which has a reduced hydrophilicity and a reduced polymer glass transition temperature (Tg) in comparison to a polyvinyl pyrrolidone (PVP) homopolymer. In the stable pharmaceutical composition of the present invention this binder is wet granulated with the moisture sensitive active pharmaceutical ingredient and one or more pharmaceutical excipients in a processing solvent to form a wet granulate before granulating the wet granulate with a second drug substance.

The stable pharmaceutical compositions comprising a moisture sensitive active pharmaceutical ingredient and a second drug substance of the present invention may further contain excipients such as tablet and capsule fillers and diluents (such as microcrystalline cellulose, lactose, starch and tri-basic calcium phosphate), disintegrants (such as starch, croscarmellose sodium, crospovidone and sodium starch glycolate), and glidants (such as colloidal silicon dioxide and talc), lubricants (such as magnesium stearate, sodium lauryl sulfate, stearic acid and sodium stearyl fumarate).

More particularly, suitable diluents and fillers for use in the pharmaceutical composition of the present invention include microcrystalline cellulose (e.g. Avicel^{®}), lactose, starch, pregelatinized starch, calcium carbonate, calcium sulfate, sugar, dextrates, dextrin, dextrose, dibasic calcium phosphate dihydrate, tribasic calcium phosphate, magnesium carbonate, magnesium oxide, maltodextrin, mannitol, powdered cellulose, sodium chloride, sorbitol and talc.

Preferably, the pharmaceutical composition of the present invention includes lactose monohydrate, more preferably in an amount of about 50% to about 65%, most preferably of about 55% to about 60% by total weight of the composition.

In an alternative preferred embodiment, the pharmaceutical composition of the present invention includes talc, more preferably in an amount of about 1 % to about 2% by total weight of the composition.

The pharmaceutical composition may also include both lactose monohydrate and talc in the amounts specified above.

Solid pharmaceutical compositions of the present invention that are compacted into a dosage form, such as a tablet, may include the addition of a disintegrant to the composition. Disintegrants include croscarmellose sodium (e.g. Ac Di Sol^{®}, Primellose^{®}), crospovidone (e.g. Kollidon^{®}, Polyplasdone^{®}), microcrystalline cellulose, polacrilin potassium, powdered cellulose, pregelatinized starch, sodium starch glycolate (e.g. Explotab^{®}, Primoljel^{®}) and starch.

Preferably, the pharmaceutical composition of the present invention includes starch, more preferably in an amount of about 20% to about 30%, most preferably about 25% by total weight of the composition.

Glidants can be added to improve the flowability of a solid composition before compaction and to improve the accuracy of dosing especially during compaction and capsule filling. Excipients that may function as glidants include colloidal silicon dioxide, magnesium trisilicate, powdered cellulose, and talc.

A lubricant can be added to the composition to reduce adhesion and/or ease the release of the product from e.g. the dye. Lubricants include magnesium stearate, calcium stearate, glyceryl monostearate, glyceryl palmitostearate, hydrogenated castor oil, hydrogenated vegetable oil, mineral oil, polyethylene glycol, sodium lauryl sulfate, sodium stearyl fumarate, stearic acid, talc and zinc stearate.

Preferably, the pharmaceutical composition of the present invention includes sodium stearyl fumarate, more preferably in an amount of about 0.5% to about 1.5%, most preferably about 1 % by total weight of the composition.

Other excipients that may be incorporated into the formulation include preservatives, surfactants, antioxidants, or any other excipient commonly used in the pharmaceutical industry.

In a preferred embodiment of the present invention, the formulation comprises Cilazapril, HCTZ, copovidone, lactose monohydrate, sodium starch glycolate, talc extra fine and sodium stearyl fumarate. Preferably, the pharmaceutical composition comprises (by total weight of the composition) Cilazapril in an amount of about 0.5% to about 15%, more preferably of about 0.6% to about 2.7%, HCTZ in an amount of about 3% to about 15%, more preferably of about 5% to about 10%, lactose monohydrate in an amount of about 50% to about 65%, more preferably of about 55% to about 60%, talc in an amount of about 1% to about 2%, starch in an amount of about 20% to about 30%, more preferably about 25%, a binder, preferably copovidone, in an amount of about 4% to about 20%, more preferably 5% to 10%, and sodium stearyl fumarate in an amount of about 0.5% to about 1.5%, more preferably about 1%.

The solid compositions of the present invention include powders, granulates, aggregates and compacted compositions. The dosages include dosages suitable for oral, buccal, and rectal administration. Although the most suitable administration in any given case will depend on the nature and severity of the condition being treated, the most preferred route of the present invention is oral. The dosages may be conveniently presented in unit dosage form and prepared by any of the methods well known in the pharmaceutical arts.

The pharmaceutical composition of the present invention may be prepared in any dosage form such as a compressed granulate in the form of a tablet for example. Also, uncompressed granulates and powder mixes that are obtained by the method of the present invention in the pre-compression steps can be simply provided in a dosage form of a capsule or sachet. Therefore, dosage forms of the pharmaceutical composition of the present invention include solid dosage forms like tablets, powders, capsules, sachets, etc. The dosage form of the present invention may also be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

Once a solid composition comprising a moisture sensitive active pharmaceutical ingredient, preferably Cilazapril, and a second drug substance, preferably HCTZ, is prepared in accordance with the present invention, it is preferably formulated into pharmaceutical formulations such as conventional dosage forms, including tablets and capsules. Tablets are a preferred dosage form. In addition, the tablets may be coated with an optional cosmetic tablet coating. More preferably this cosmetic coat has "moisture barrier" properties. This moisture barrier property provides protection against environmental moisture for sensitive cores, enhances product stability, and improves shelf life. Preferably, the cosmetic coating is a tablet coating based on polyvinyl alcohol. More preferably, the cosmetic coating comprises polyvinyl alcohol, talc and polyethylene glycol (PEG). Most preferably, the cosmetic coating further comprises an opacifier and/or a colorant, e.g. titanium dioxide and/or iron oxide.

The commercially available series of powder mixes for coating suspension sold as the Opadry^{®}II 85F series (a coating with moisture barrier properties), available from Colorcon, which are based on Polyvinyl alcohol, are examples of such cosmetic coat. In addition to Polyvinyl Alcohol, this Opadry series of products comprise Talc, PEG 3350, Titanium Dioxide and pigments. Preferably, the tablets of the present invention comprises a cosmetic coat of about 2% to about 6% of the tablet weight, more preferably of about 2.5% to about 4.5% of the tablet weight, most preferably of about 3% to about 3.5% of the tablet weight.

In another embodiment the present invention provides a method of preparing a pharmaceutical composition comprising a moisture sensitive active pharmaceutical ingredient and a second active pharmaceutical ingredient comprising the following steps of:
a) providing a moisture sensitive active pharmaceutical ingredient;
b) mixing the moisture sensitive active pharmaceutical ingredient with at least one pharmaceutically acceptable excipient, forming a mixture; and
c) wet granulating the mixture with a solution of a binder excipient dissolved in one or more processing solvents forming a wet granulate;
d) providing a material comprising a second active pharmaceutical ingredient and optionally one or more pharmaceutical excipients; and
e) adding the material from step d) to the wet granulate from step c) forming a combined granulate,
wherein when the material of step d) comprises a second pharmaceutical ingredient and one or more pharmaceutical excipients the material is optionally a mixture obtained by mixing the second pharmaceutical ingredient with the one or more pharmaceutical excipients.

Preferably, the moisture sensitive active pharmaceutical ingredient is Cilazapril and the second active pharmaceutical ingredient is Hydrochlorothiazide (HCTZ).

In a preferred embodiment, the pharmaceutical excipient(s) employed in step b) do not include a binder.

Preferably the amount of the moisture sensitive active pharmaceutical ingredient in the composition is about 0.1% to about 25%, more preferably of about 0.5% to about 15%, of the total weight of the composition. A most preferred amount of the moisture sensitive active pharmaceutical ingredient in the composition is about 0.6% to about 2.7% of the total weight of the composition. Preferably, the moisture sensitive active pharmaceutical ingredient is Cilazapril.

Preferably the amount of the second active pharmaceutical ingredient in the composition is about 1% to about 25%, more preferably of about 3% to about 15%, of the total weight of the composition. A most preferred amount of the second active pharmaceutical ingredient in the composition is about 5% to about 10% of the total weight of the composition. Preferably, when the moisture sensitive active pharmaceutical ingredient is an ACE inhibitor, the second active pharmaceutical ingredient is Hydrochlorothiazide (HCTZ).

In preparing a pharmaceutical composition of the present invention a typical granulation process involves mixing the moisture sensitive active ingredient and possibly excipients in a mixer. The binder is dissolved in the processing solvent used for granulating although a further portion of binder or another binder may be one of the excipients added in the initial dry mix state with the moisture sensitive drug substance. The granulating/processing solvent, solution or suspension is added to the dry powders in the mixer and mixed to form a wet granulate. The second drug substance is added to the wet granulate either alone or with one or more excipients, optionally in a processing solvent, and mixed until the desired characteristics are achieved. This usually produces a granule that will have suitable characteristics for producing tablets with adequate hardness, dissolution, content uniformity, and other physical characteristics. After the wet granulation step, the product is most often dried and then milled after drying, to obtain a major percentage of the product within a desired size range. Preferably, the product after wet granulation is dried until the loss on drying (LOD) is not more than about 2.5%, more preferably not more than about 1.5%. Preferably, the product is milled or sized through a 1mm aperture screen, more preferably through a 0.8mm aperture screen.

Preferably, the stable pharmaceutical composition of the present invention is prepared by wet granulation with a suitable solvent/processing solvent. A suitable solvent/processing solvent is able to dissolve the selected binder. Preferably, the solvent/processing solvent is capable of dissolving the binder to reach a concentration of at least about 10% W/W. More preferably, the solvent/processing solvent is selected from the group consisting of ethanol, isopropyl alcohol, water, and combinations thereof. Preferably, the stable formulation prepared by wet granulation comprises at least 4%, preferably about 4% to about 20%, more preferably about 5% to about 10%, of a binder by weight of the formulation. Suitable binders for use in the method of the present invention include cellulose derivatives, polyvinyl pyrrolidones (PCP) and its derivatives, polyvinylacetates (PVA), or polyvinylalcohols. Preferably, the binder comprises at least Copovidone and more preferably, the binder is applied as a solution in ethanol or water. A preferred solution of the binder in ethanol or water comprises about 25% to about 55% (w/w) binder, preferably Copovidone, more preferably about 30% to about 50% (w/w) binder, preferably Copovidone.

Moreover, the co-pending European patent application No. 06252877.3, filed June 2, 2006, incorporated herein by reference, further describes preparing a stable pharmaceutical composition comprising a moisture sensitive active pharmaceutical ingredient using wet granulation. A process of preparing a wet granulate comprising a moisture sensitive active pharmaceutical ingredient and a binder is described therein.

The method of the present invention may further comprise steps in preparing a tablet or capsule of the pharmaceutical composition of the present invention. In preparing such tablet the method further comprises the steps of
f) mixing the combined granulate from step f) with one or more excipients forming a final blend;
g) pressing the final blend into a tablet; and
h) optionally coating the tablet with a cosmetic coat. Preferably, the cosmetic coat has moisture barrier properties. Examples of such cosmetic coatings are tablet coatings based on polyvinyl alcohol.

The optional cosmetic coating of the tablet preferably comprises preparing a suspension comprising about 10% to about 25%, preferably about 12% to about 15%, more preferably about 12% to about 13%, of a powder mixture for cosmetic coating, and applying the suspension on the tablet. The cosmetic coating suspension is preferably prepared such that the tablet comprises about 2% to about 6%, preferably about 2.5% to about 4.5%, of a tablet cosmetic coat. The tablet cosmetic coat in the present invention preferably has "moisture barrier" properties. The commercially available series of powder mixes for coating suspension sold as the Opadry^{®}II 85F series, available from Colorcon, which are based on Polyvinyl alcohol, are examples of such cosmetic coat with moisture barrier properties.

Capsules comprising either a hard or soft shell and containing the composition of the present invention may be prepared. The shell may be made from gelatin and optionally contain a plasticizer such as glycerin and sorbitol, and an opacifying agent or colorant. A capsule filling of the present invention may comprise the granulates that were described with reference to tableting, a final blend of a granulate composition of the present invention mixed with one or more excipients, however they are not subjected to a final tableting step. Further, such capsules may be prepared by any of the methods well known in the pharmaceutical arts.

Further, in a preferred embodiment, the present invention provides a method for preparing a stable pharmaceutical composition comprising:
a) mixing cilazapril, lactose, talc and sodium starch glycolate;
b) adding a solution of a binder, preferably copovidone, to the mixture obtained in step a) to form a wet granulate;
c) optionally combining the wet granulate with further starch and mixing;
d) adding HCTZ to the wet granulate and mixing;
e) drying and then milling the granulate; and
f) adding sodium stearyl fumarate to the granulate obtained in step e) and mixing to obtain a final blend.

The compositions of the present invention are useful in therapy. In particular, the compositions of the invention are useful in treating hypertension.

The present invention also provides a method of treating a patient suffering from a disease, preferably hypertension, comprising administering to a patient in need thereof a therapeutically effective amount of a stable pharmaceutical composition comprising a moisture sensitive active pharmaceutical ingredient, preferably Cilazapril, a second active pharmaceutical ingredient, preferably Hydrochlorothiazide, and at least one pharmaceutical excipient, wherein the active pharmaceutical ingredients are wet granulated with a solution of at least one pharmaceutical excipient.

The following examples are presented in order to further illustrate the invention. These examples should not be construed in any manner to limit the invention.

### EXAMPLES

### Example 1. Wet granulation, Hypromellose (HPMC) as a binder, one step granulation

In a high shear mixer were mixed for 1 minute: 7.8 g of Cilazapril Monohydrate, 178.8 g of Lactose Monohydrate, 4.5 g of Talc Extra Fine, 18.8 g of Hydrochlorothiazide and 75.0 g of Starch. 60 g of a 20% (w/w) aqueous solution of Hypromellose was added and the mass was mixed in the high shear mixer for 4 minutes. 6 g of water was added and the blend was mixed for 1 minute in the high shear mixer. The obtained granulate was dried using a fluid bed dryer and the dry granulate was milled in an oscillating granulator through 0.8 mm screen. The milled granulate was combined with 2.7 g of screened Sodium Stearyl Fumarate and mixed in a Y-cone blender for 5 minutes.

Tablets were pressed from the final blend in a rotary tablet press. The tablets were packed in cold formed aluminium blister covered with aluminium foil. Packed tablets were stored at 55°C. The main degradation product, Cilazaprilat, was tested using HPLC method.

### Example 2. Wet granulation, Copovidone as a binder, one step granulation

In a high shear mixer were mixed for 1 minute: 5.2 g of Cilazapril Monohydrate, 115.3 g of Lactose Monohydrate, 3.0 g of Talc Extra Fine, 12.5 g of Hydrochlorothiazide and 50.0 g of Starch. 33 g of a 36.4% (w/w) aqueous solution of Copovidone was added and the mass was mixed in the high shear mixer for 3 minutes. The obtained granulate was dried using a fluid bed dryer and the dry granulate was milled in an oscillating granulator through 0.8 mm screen. The milled granulate was combined with 1.6 g of screened Sodium Stearyl Fumarate and mixed in a Y-cone blender for 5 minutes.

Tablets were pressed from the final blend in a rotary tablet press. The tablets were packed in cold formed aluminium blister covered with aluminium foil. Packed tablets were stored at 55°C. The main degradation product, Cilazaprilat, was tested using HPLC method.

### Example 3. Wet granulation, Hypromellose (HPMC) as a binder, two steps granulation

In a high shear mixer were mixed for 1 minute: 10.4 g of Cilazapril Monohydrate, 238.4 g of Lactose Monohydrate, 6.0 g of Talc Extra Fine, and 100.0 g of Starch. 80 g of a 20% (w/w) aqueous solution of Hypromellose was added and the mass was mixed in the high shear mixer for 3 minutes. 8.2 g of water was added and the blend was mixed for 1 minute in the high shear mixer. 25.0 g of Hydrochlorothiazide was added to the wet blend and the mass was mixed for 2 minutes in the high shear mixer. The obtained granulate was dried using a fluid bed dryer and the dry granulate was milled in an oscillating granulator through 0.8 mm screen. The milled granulate was combined with 3.8 g of screened Sodium Stearyl Fumarate and mixed in a Y-cone blender for 5 minutes.

Tablets were pressed from the final blend in a rotary tablet press. The tablets were packed in cold formed aluminium blister covered with aluminium foil. Packed tablets were stored at 55°C. The main degradation product, Cilazaprilat, was tested using HPLC method.

### Example 4. Wet granulation, Copovidone as a binder, two steps granulation

In a high shear mixer were mixed for 1 minute: 10.4 g of Cilazapril Monohydrate, 234.4 g of Lactose Monohydrate, 6.0 g of Talc Extra Fine, and 100.0 g of Starch. 66 g of a 36.4% (w/w) aqueous solution of Copovidone was added and the mass was mixed in the high shear mixer for 10 minutes. 25.0 g of Hydrochlorothiazide was added to the wet blend and the mass was mixed for 2 minutes in the high shear mixer. The obtained granulate was dried using a fluid bed dryer and the dry granulate was milled in an oscillating granulator through 0.8 mm screen. The milled granulate was combined with 3.9 g of screened Sodium Stearyl Fumarate and mixed in a Y-cone blender for 5 minutes.

Tablets were pressed from the final blend in a rotary tablet press.
The tablets were packed in cold formed aluminium blister covered with aluminium foil. Packed tablets were stored at 55 ° C. The main degradation product, Cilazaprilat, was tested using HPLC method.

### Example 5. Wet granulation, Copovidone as a binder, two steps granulation, starch and part of the granulation solution added in second granulation step.

In a high shear mixer were mixed for 1 minute: 5.2 g of Cilazapril Monohydrate, 115.3 g of Lactose Monohydrate and 3.0 g of Talc Extra Fine. 27.5 g of a 36.4% (w/w) aqueous solution of Copovidone was added and the mass was mixed in the high shear mixer for 1.7 minutes. 50.0 g of Starch was added to the wet blend and the mass was mixed for 2 minutes in the high shear mixer.12.5 g of Hydrochlorothiazide was added to the wet blend and the mass was mixed for 20 second in the high shear mixer. 5.5 g of 36.4% (w/w) aqueous solution of Copovidone was added and the mass was mixed in the high shear mixer for 2 minutes. The obtained granulate was dried using a fluid bed dryer and the dry granulate was milled in an oscillating granulator through 0.8 mm screen. The milled granulate was combined with 1.7 g of screened Sodium Stearyl Fumarate and mixed in a Y-cone blender for 5 minutes.

Tablets were pressed from the final blend in a rotary tablet press. The tablets were packed in cold formed aluminium blister covered with aluminium foil. Packed tablets were stored at 55°C. The main degradation product, Cilazaprilat, was tested using HPLC method.

**Table 2. Examples and comparative examples of pharmaceutical compositions comprising Cilazapril, a moisture sensitive active pharmaceutical ingredient, and Hydrochlorothiazide.**

| **Ex. 5** | **Ex. 4** | **Ex. 2** | **Ex. 1 and 3** | **Example** |
|---|---|---|---|---|
| **Copovidone** | **Copovidone** | **Copovidone** | **HPMC** | **Binder** |
| **Content, % of the end tablet weight** | | | | **Ingredient** |
| 2.61 | 2.61 | 2.58 | 2.61 | Cilazapril Monohydrate |
| 6.25 | 6.25 | 6.19 | 6.25 | Hydrochlorothiazide |
| 57.64 | 57.64 | 58.01 | 59.59 | Lactose Monohydrate |
| 1.50 | 1.50 | 1.49 | 1.50 | Talc Extra Fine |
| 25.00 | 25.00 | 24.75 | 25.00 | Starch |
| | | | 4.00 | Hypromellose |
| 6.00 | 6.00 | 5.94 | | Copovidone |
| 1.00 | 1.00 | 1.04 | 1.05 | Sodium Stearyl Fumarate |
| 10.5 | 10.5 | 10.5 | 22.2/18.2 | Water (process solvent only) |

### Example 6. Stability testing of pharmaceutical compositions of Cilazapril and HCTZ.

Comparative stability tests were performed comparing the stability of pharmaceutical compositions of Cilazapril and HCTZ prepared either in a process wet granulating a mixture of Cilazapril and HCTZ (a "single step wet granulation process") with such pharmaceutical compositions prepared in a process wet granulating first Cilazapril forming a wet granulate to which the HCTZ is added (a "two step wet granulation process"). Table 3 shows the results of storage of such compositions (Examples 1-5) under stress conditions of 55°C for 4 weeks. The results in the table shows that the two step wet granulation process of pharmaceutical compositions of Cilazapril and HCTZ of the present invention provides a stable pharmaceutical composition compared to the one step wet granulating process.

**Table 3. Degradation under "stress" conditions (55 ° C, storage period 4 weeks), of Cilazapril and HCTZ displayed as function of different formulations and manufacturing methods.**

| **Two steps granulation, Copovidone as a binder (ex. 5)** | **Two steps granulation, Copovidone as a binder (ex. 4)** | **Two steps granulation, HPMC as a binder (ex. 3)** | **One step granulation, Copovidone as a binder (ex. 2)** | **One step granulation, HPMC as a binder (ex. 1)** | **Description** |
|---|---|---|---|---|---|
| 0.1 | 0.1 | 0.1 | 0.2 | 0.3 | **Major degradation product, Cilazaprilat, Time "Zero". % per labeled claim of Cilazapril** |
| 1.4 | 1.3 | 2.4 | 8.3 | 8.8 | **Major degradation product, Cilazaprilat, storage period 4 weeks, % per labeled claim of Cilazapril** |

## Claims

1. A pharmaceutical composition comprising;
a) a moisture sensitive active pharmaceutical ingredient; and
b) a second active pharmaceutical ingredient;
wherein the moisture sensitive active pharmaceutical ingredient is first wet granulated with a solution of at least one pharmaceutical excipient in at least one processing solvent before granulation with the second active pharmaceutical ingredient.

2. The pharmaceutical composition according to claim 1, wherein the amount of the moisture sensitive active pharmaceutical ingredient is about 0.1% to about 25% of the total weight of the composition.

3. The pharmaceutical composition according to claim 2, wherein the amount of the moisture sensitive active pharmaceutical ingredient is about 0.5% to about 15% of the total weight of the composition.

4. The pharmaceutical composition according to claim 3, wherein the amount of the moisture sensitive active pharmaceutical ingredient is about 0.6% to about 2.7% of the total weight of the composition.

5. The pharmaceutical composition according to any one of the preceding claims, wherein the moisture sensitive active pharmaceutical ingredient is Cilazapril.

6. The pharmaceutical composition according to any one of the preceding claims, wherein the amount of the second active pharmaceutical ingredient is about 1% to about 25% of the total weight of the composition.

7. The pharmaceutical composition according to claim 6, wherein the amount of the second active pharmaceutical ingredient is about 6% to about 10% of the total weight of the composition.

8. The pharmaceutical composition according to any one of the preceding claims, wherein the second active pharmaceutical ingredient is Hydrochlorothiazide.

9. The pharmaceutical composition according to any one of the preceding claims, wherein at least one pharmaceutical excipient is a binder.

10. The pharmaceutical composition according to claim 9, wherein the binder is selected from the group consisting of cellulose derivatives, polyvinyl pyrrolidones and their derivatives, polyvinyl acetates, and polyvinyl alcohols.

11. The pharmaceutical composition according to claim 10, where the binder is selected from the group consisting of Copovidone and Hypromellose.

12. The pharmaceutical composition according to any one of claims 9 to 11, wherein the amount of the binder is at least about 4% of the total weight of the composition.

13. The pharmaceutical composition according to claim 12, wherein the amount of the binder is about 4% to about 20% of the total weight of the composition.

14. The pharmaceutical composition according to claim 13, wherein the amount of the binder is about 5% to about 10% of the total weight of the composition.

15. The pharmaceutical composition according to any one of the preceding claims, wherein the moisture sensitive active pharmaceutical ingredient has a major degradation product and wherein the composition comprises this major degradation product in an amount not more than about 3% by weight of the total initial weight of the moisture sensitive active pharmaceutical ingredient in the pharmaceutical composition after storage.

16. The pharmaceutical composition according to claim 15, where in the amount of the major degradation product of the moisture sensitive active pharmaceutical ingredient in the pharmaceutical composition is not more than about 2% by weight of the total initial weight of the moisture sensitive active pharmaceutical ingredient.

17. The pharmaceutical composition according to claim 16, where in the amount of the major degradation product of the moisture sensitive active pharmaceutical ingredient in the pharmaceutical composition is not more than about 1% by weight of the total initial weight of the moisture sensitive active pharmaceutical ingredient.

18. The pharmaceutical composition according to any one of claims 15 to 17, wherein storage is in a package with moisture barrier properties, which are at least as efficient as aluminum-aluminum cold form blisters.

19. The pharmaceutical composition according to claim 18, wherein storage is at 55°C for four weeks.

20. The pharmaceutical composition according to any one of the preceding claims, wherein the composition is in a solid dosage form.

21. The pharmaceutical composition according to claim 20, wherein the dosage form is selected from the group consisting of a tablet and a capsule.

22. The pharmaceutical composition according to claim 21, wherein the dosage form is a tablet.

23. The pharmaceutical composition according to claim 22, wherein the tablet comprises a cosmetic tablet coating.

24. The pharmaceutical composition according to claim 23, wherein the cosmetic tablet coating has moisture barrier properties.

25. The pharmaceutical composition according to claim 24, wherein the cosmetic tablet coating having moisture barrier properties is selected from the group consisting of the Opadry® 85F series tablet coatings.

26. The pharmaceutical composition according to any one of claims 23 to 25, wherein the cosmetic tablet coating is in an amount of about 2% to about 6% of the tablet weight.

27. The pharmaceutical composition according to claim 26, wherein the amount of the cosmetic tablet coating is about 3% to about 3.5% of the tablet weight.

28. A method of preparing a pharmaceutical composition comprising a moisture sensitive active pharmaceutical ingredient and a second active pharmaceutical ingredient comprising the following steps of:
a) providing a moisture sensitive active pharmaceutical ingredient;
b) mixing the moisture sensitive active pharmaceutical ingredient with at least one pharmaceutically acceptable excipient, forming a mixture; and
c) wet granulating the mixture with a solution of a binder excipient dissolved in one or more processing solvents forming a wet granulate;
d) providing a material comprising a second active pharmaceutical ingredient and optionally one or more pharmaceutical excipients; and
e) adding the material from step d) to the wet granulate from step c) forming a combined granulate,
wherein when the material of step d) comprises a second pharmaceutical ingredient and one or more pharmaceutical excipients the material is optionally a mixture obtained by mixing the second pharmaceutical ingredient with the one or more pharmaceutical excipients.

29. The method according to claim 28, wherein the amount of the moisture sensitive active pharmaceutical ingredient is about 0.1 % to about 25% and the amount of the second active pharmaceutical ingredient is about 1% to about 25% of the total weight of the composition.

30. The method according to claim 29, wherein the amount of the moisture sensitive active pharmaceutical ingredient is about 0.6% to about 2.7% and the amount of the second active pharmaceutical ingredient is about 5% to about 10% of the total weight of the composition.

31. The method according to any one of claims 28 to 30, wherein the moisture sensitive active pharmaceutical ingredient is Cilazapril and the second pharmaceutical ingredient is Hydrochlorothiazide.

32. The method according to any one of claims 28 to 31, wherein the binder is selected from the group consisting of cellulose derivatives, polyvinyl pyrrolidones and their derivatives, polyvinyl acetates, and polyvinyl alcohols.

33. The method according to claim 32, wherein the binder is selected from the group consisting of Copovidone and Hypromellose.

34. The method according to any one of claims 28 to 33, wherein the amount of the binder is at least about 4% of the total weight of the composition.

35. The method according to claim 34, wherein the amount of the binder is about 5% to about 10% of the total weight of the composition.

36. The method according to any one of claims 28 to 35, wherein the processing solvent is selected from the group consisting of ethanol, isopropanol, water, and combinations thereof.

37. The method according to any one of claims 28 to 36, wherein the binder is applied as a solution in water or ethanol.

38. The method according to claim 37, wherein the solution of the binder in water or ethanol comprises about 25% to about 55% (w/w) of the binder.

39. The method according to claim 38, wherein the solution of the binder in water or ethanol comprises about 30% to about 50% (w/w) of the binder.

40. The method according to any one of claims 28 to 39 in preparing a pharmaceutical composition, wherein the method further comprises the steps of:
f) mixing the combined granulate from step e) with one or more excipients forming a final blend;
g) pressing the final blend into a tablet; and
h) optionally coating the tablet with a cosmetic coat.

41. The method according to claim 40, wherein the step of coating the tablet comprises preparing a suspension comprising about 10% to about 15% of a powder mixture for cosmetic coating, and applying the suspension on the tablet.

42. The method according to claim 41, wherein the suspension comprises about 12% to about 13% of a powder mixture for cosmetic coating.

43. The method according to claim 41 or claim 42, wherein the cosmetic coat has moisture barrier properties and the powder mixture for cosmetic coating is selected from the powder mixtures of the Opadry® 85F series.

44. The method according to any one of claims 28 to 39 in preparing a pharmaceutical composition, wherein the method further comprises mixing the granulate with one or more excipients forming a final blend and filling the final blend in a capsule.

45. The method according to any one of claims 28 to 44, wherein the pharmaceutically acceptable excipient(s) employed in step b) do not include a binder.

46. A pharmaceutical composition obtainable by a method as defined in any one of claims 28 to 45.

47. A pharmaceutical composition as defined in any one of claims 1 to 27 for use in therapy.

48. A pharmaceutical composition as defined in any one of claims 1 to 27 for treating hypertension.

49. Use of cilazapril in the preparation of a pharmaceutical composition as defined in any one of claims 1 to 27 for treating hypertension.

## Patentansprüche

1. Pharmazeutische Zusammensetzung, welche folgendes umfaßt:
a) einen feuchtigkeitsempfindlichen aktiven pharmazeutischen Inhaltsstoff und
b) einen zweiten aktiven pharmazeutischen Inhaltsstoff,
wobei der feuchtigkeitsempfindliche aktive pharmazeutische Inhaltsstoff zuerst mit einer Lösung von wenigstens einem pharmazeutischen Hilfsstoff in wenigstens einem Verarbeitungslösungsmittel naßgranuliert wird, ehe Granulation mit dem zweiten aktiven pharmazeutischen Inhaltsstoff erfolgt.

2. Pharmazeutische Zusammensetzung nach Anspruch 1, wobei die Menge des feuchtigkeitsempfindlichen aktiven pharmazeutischen Inhaltsstoffs etwa 0, 1 % bis etwa 25% des Gesamtgewichts der Zusammensetzung ausmacht.

3. Pharmazeutische Zusammensetzung nach Anspruch 2, wobei die Menge des feuchtigkeitsempfindlichen aktiven pharmazeutischen Inhaltsstoffs etwa 0,5% bis etwa 15% des Gesamtgewichts der Zusammensetzung ausmacht.

4. Pharmazeutische Zusammensetzung nach Anspruch 3, wobei die Menge des feuchtigkeitsempfindlichen aktiven pharmazeutischen Inhaltsstoffs etwa 0,6% bis etwa 2,7% des Gesamtgewichts der Zusammensetzung ausmacht.

5. Pharmazeutische Zusammensetzung nach einem der vorangegangenen Ansprüche, wobei der feuchtigkeitsempfindliche aktive pharmazeutische Inhaltsstoff Cilazapril ist.

6. Pharmazeutische Zusammensetzung nach einem der vorangegangenen Ansprüche, wobei die Menge des zweiten aktiven pharmazeutischen Inhaltsstoffs etwa 1% bis etwa 25% des Gesamtgewichts der Zusammensetzung ausmacht.

7. Pharmazeutische Zusammensetzung nach Anspruch 6, wobei die Menge des zweiten aktiven pharmazeutischen Inhaltsstoffs etwa 6% bis etwa 10% des Gesamtgewichts der Zusammensetzung ausmacht.

8. Pharmazeutische Zusammensetzung nach einem der vorangegangenen Ansprüche, wobei der zweite aktive pharmazeutische Inhaltsstoff Hydrochlorthiazid ist.

9. Pharmazeutische Zusammensetzung nach einem der vorangegangenen Ansprüche, wobei wenigstens ein pharmazeutischer Hilfsstoff ein Bindemittel ist.

10. Pharmazeutische Zusammensetzung nach Anspruch 9, wobei das Bindemittel aus der Gruppe ausgewählt ist, bestehend aus Zellulosederivaten, Polyvinylpyrrolidonen und deren Derivaten, Polyvinylacetaten und Polyvinylalkoholen.

11. Pharmazeutische Zusammensetzung nach Anspruch 10, wobei das Bindemittel aus der Gruppe ausgewählt ist, bestehend aus Copovidon und Hypromellose.

12. Pharmazeutische Zusammensetzung nach einem der Ansprüche 9 bis 11, wobei die Menge des Bindemittels wenigstens etwa 4% des Gesamtgewichts der Zusammensetzung ausmacht.

13. Pharmazeutische Zusammensetzung nach Anspruch 12, wobei die Menge des Bindemittels etwa 4% bis etwa 20% des Gesamtgewichts der Zusammensetzung ausmacht.

14. Pharmazeutische Zusammensetzung nach Anspruch 13, wobei die Menge des Bindemittels etwa 5% bis etwa 10% des Gesamtgewichts der Zusammensetzung ausmacht.

15. Pharmazeutische Zusammensetzung nach einem der vorangegangenen Ansprüche, wobei der feuchtigkeitsempfindliche aktive pharmazeutische Inhaltsstoff ein Hauptabbauprodukt hat und wobei die Zusammensetzung dieses Hauptabbauprodukt in einer Menge von nicht mehr als etwa 3 Gewichts-% des gesamten anfänglichen Gewichts des feuchtigkeitsempfindlichen aktiven pharmazeutischen Inhaltsstoffs in der pharmazeutischen Zusammensetzung nach Lagerung umfaßt.

16. Pharmazeutische Zusammensetzung nach Anspruch 15, wobei die Menge des Hauptabbauprodukts des feuchtigkeitsempfindlichen aktiven pharmazeutischen Inhaltsstoffs in der pharmazeutischen Zusammensetzung nicht mehr als etwa 2 Gewichts-% des gesamten anfänglichen Gewichts des feuchtigkeitsempfindlichen aktiven pharmazeutischen Inhaltsstoffs ausmacht.

17. Pharmazeutische Zusammensetzung nach Anspruch 16, wobei die Menge des Hauptabbauprodukts des feuchtigkeitsempfindlichen aktiven pharmazeutischen Inhaltsstoffs in der pharmazeutischen Zusammensetzung nicht mehr als etwa 1 Gewichts-% des gesamten anfänglichen Gewichts des feuchtigkeitsempfindlichen aktiven pharmazeutischen Inhaltsstoffs ausmacht.

18. Pharmazeutische Zusammensetzung nach einem der Ansprüche 15 bis 17, wobei die Lagerung in einer Verpackung mit Feuchtigkeitsbarriereeigenschaften, die wenigstens so effektiv sind wie Aluminium-Aluminium-Kaltformblister, erfolgt.

19. Pharmazeutische Zusammensetzung nach Anspruch 18, wobei die Lagerung bei 55°C für vier Wochen erfolgt.

20. Pharmazeutische Zusammensetzung nach einem der vorangegangenen Ansprüche, wobei die Zusammensetzung in fester Dosierungsform vorliegt.

21. Pharmazeutische Zusammensetzung nach Anspruch 20, wobei die Dosierungsform aus der Gruppe ausgewählt ist, bestehend aus einer Tablette und einer Kapsel.

22. Pharmazeutische Zusammensetzung nach Anspruch 21, wobei die Dosierungsform eine Tablette ist.

23. Pharmazeutische Zusammensetzung nach Anspruch 22, wobei die Tablette eine kosmetische Tablettenbeschichtung umfaßt.

24. Pharmazeutische Zusammensetzung nach Anspruch 23, wobei die kosmetische Tablettenbeschichtung Feuchtigkeitsbarriereeigenschaften besitzt.

25. Pharmazeutische Zusammensetzung nach Anspruch 24, wobei die kosmetische Tablettenbeschichtung mit Feuchtigkeitsbarriereeigenschaften aus der Gruppe ausgewählt ist, bestehend aus Tablettenbeschichtungen der Opadry^{®} 85F-Serie.

26. Pharmazeutische Zusammensetzung nach einem der Ansprüche 23 bis 25, wobei die kosmetische Tablettenbeschichtung in einer Menge von etwa 2% bis etwa 6% des Tablettengewichts vorliegt.

27. Pharmazeutische Zusammensetzung nach Anspruch 26, wobei die Menge der kosmetischen Tablettenbeschichtung etwa 3% bis etwa 3,5% des Tablettengewichts ausmacht.

28. Verfahren zum Herstellen einer pharmazeutischen Zusammensetzung, umfassend einen feuchtigkeitsempfindlichen aktiven pharmazeutischen Inhaltsstoff und einen zweiten aktiven pharmazeutischen Inhaltsstoff, wobei das Verfahren die folgenden Stufen umfaßt:
a) Bereitstellen eines feuchtigkeitsempfindlichen aktiven pharmazeutischen Inhaltsstoffs,
b) Mischen des feuchtigkeitsempfindlichen aktiven pharmazeutischen Inhaltsstoffs mit wenigstens einem pharmazeutisch verträglichen Hilfsstoff unter Bildung eines Gemischs und
c) Naßgranulieren des Gemischs mit einer Lösung aus einem Bindemittelhilfsstoff, gelöst in einem oder mehreren Verarbeitungslösungsmitteln, unter Bildung eines nassen Granulats,
d) Bereitstellen eines Materials, welches einen zweiten aktiven pharmazeutischen Inhaltsstoff und optional einen oder mehrere pharmazeutische Hilfsstoffe umfaßt, und
e) Zugeben des Materials aus Stufe d) zu dem nassen Granulat aus Stufe c) unter Bildung eines vereinigten Granulats,
wobei, wenn das Material aus Stufe d) einen zweiten pharmazeutischen Inhaltsstoff und einen oder mehrere pharmazeutische Hilfsstoffe umfaßt, das Material optional ein Gemisch ist, welches durch Mischen des zweiten pharmazeutischen Inhaltsstoffs mit dem einen oder den mehreren pharmazeutischen Hilfsstoffen erhalten wird.

29. Verfahren nach Anspruch 28, wobei die Menge des feuchtigkeitsempfindlichen aktiven pharmazeutischen Inhaltsstoffs etwa 0,1% bis etwa 25% und die Menge des zweiten aktiven pharmazeutischen Inhaltsstoffs etwa 1 % bis etwa 25% des Gesamtgewichts der Zusammensetzung ausmachen.

30. Verfahren nach Anspruch 29, wobei die Menge des feuchtigkeitsempfindlichen aktiven pharmazeutischen Inhaltsstoffs etwa 0,6% bis etwa 2,7% und die Menge des zweiten aktiven pharmazeutischen Inhaltsstoffs etwa 5% bis etwa 10% des Gesamtgewichts der Zusammensetzung ausmachen.

31. Verfahren nach einem der Ansprüche 28 bis 30, wobei der feuchtigkeitsempfindliche aktive pharmazeutische Inhaltsstoff Cilazapril ist und der zweite pharmazeutische Inhaltsstoff Hydrochlorthiazid ist.

32. Verfahren nach einem der Ansprüche 28 bis 31, wobei das Bindemittel aus der Gruppe ausgewählt ist, bestehend aus Zellulosederivaten, Polyvinylpyrrolidonen und deren Derivaten, Polyvinylacetaten und Polyvinylalkoholen.

33. Verfahren nach Anspruch 32, wobei das Bindemittel aus der Gruppe ausgewählt ist, bestehend aus Copovidon und Hypromellose.

34. Verfahren nach einem der Ansprüche 28 bis 33, wobei die Menge des Bindemittels wenigstens etwa 4% des Gesamtgewichts der Zusammensetzung ausmacht.

35. Verfahren nach Anspruch 34, wobei die Menge des Bindemittels etwa 5% bis etwa 10% des Gesamtgewichts der Zusammensetzung ausmacht.

36. Verfahren nach einem der Ansprüche 28 bis 35, wobei das Verarbeitungslösungsmittel aus der Gruppe ausgewählt ist, bestehend aus Ethanol, Isopropanol, Wasser und Kombinationen davon.

37. Verfahren nach einem der Ansprüche 28 bis 36, wobei das Bindemittel als eine Lösung in Wasser oder Ethanol angewandt wird.

38. Verfahren nach Anspruch 37, wobei die Lösung des Bindemittels in Wasser oder Ethanol etwa 25% bis etwa 55% (w/w) des Bindemittels umfaßt.

39. Verfahren nach Anspruch 38, wobei die Lösung des Bindemittels in Wasser oder Ethanol etwa 30% bis etwa 50% (w/w) des Bindemittels umfaßt.

40. Verfahren nach einem der Ansprüche 28 bis 39 bei der Herstellung einer pharmazeutischen Zusammensetzung, wobei das Verfahren weiterhin die folgenden Stufen umfaßt:
f) Mischen des vereinigten Granulats aus Stufe e) mit einem oder mehreren Hilfsstoffen unter Bildung eines Endgemischs,
g) Pressen des Endgemischs zu einer Tablette und
h) optional Beschichten der Tablette mit einer kosmetischen Beschichtung.

41. Verfahren nach Anspruch 40, wobei die Stufe des Beschichtens der Tablette das Herstellen einer Suspension, umfassend etwa 10% bis etwa 15% eines Pulvergemischs für die kosmetische Beschichtung, und das Aufbringen der Suspension auf die Tablette umfaßt.

42. Verfahren nach Anspruch 41, wobei die Suspension etwa 12% bis etwa 13% eines Pulvergemischs für die kosmetische Beschichtung umfaßt.

43. Verfahren nach Anspruch 41 oder Anspruch 42, wobei die kosmetische Beschichtung Feuchtigkeitsbarriereeigenschaften besitzt und das Pulvergemisch für die kosmetische Beschichtung unter den Pulvergemischen der Cpadry^{®} 85F-Serie ausgewählt ist.

44. Verfahren nach einem der Ansprüche 28 bis 39 bei der Herstellung einer pharmazeutischen Zusammensetzung, wobei das Verfahren weiterhin das Mischen des Granulats mit einem oder mehreren Hilfsstoffen unter Bildung eines Endgemischs und das Einfüllen des Endgemischs in eine Kapsel umfaßt.

45. Verfahren nach einem der Ansprüche 28 bis 44, wobei der in Stufe b) verwendete pharmazeutische Hilfsstoff (die in Stufe b) verwendeten pharmazeutischen Hilfsstoffe) kein Bindemittel umfaßt (umfassen).

46. Pharmazeutische Zusammensetzung, erhältlich durch ein Verfahren, wie es in einem der Ansprüche 28 bis 45 definiert ist.

47. Pharmazeutische Zusammensetzung, wie sie in einem der Ansprüche 1 bis 27 definiert ist, zur Verwendung in der Therapie.

48. Pharmazeutische Zusammensetzung, wie sie in einem der Ansprüche 1 bis 27 definiert ist, zur Behandlung von Bluthochdruck.

49. Verwendung von Cilazapril bei der Herstellung einer pharmazeutischen Zusammensetzung, wie sie in einem der Ansprüche 1 bis 27 definiert ist, zur Behandlung von Bluthochdruck.

## Revendications

1. Composition pharmaceutique comprenant :
a) un principe actif pharmaceutique sensible à l'humidité ; et
b) un deuxième principe actif pharmaceutique ;
dans laquelle le principe actif pharmaceutique sensible à l'humidité est granulé par voie humide dans un premier temps avec une solution d'au moins un excipient pharmaceutique dans au moins un solvant de traitement avant granulation avec le deuxième principe actif pharmaceutique.

2. Composition pharmaceutique selon la revendication 1, dans laquelle la quantité du principe actif pharmaceutique sensible à l'humidité est d'environ 0, 1 % à environ 25% par rapport au poids total de la composition.

3. Composition pharmaceutique selon la revendication 2, dans laquelle la quantité du principe actif pharmaceutique sensible à l'humidité est d'environ 0,5% à environ 15% par rapport au poids total de la composition.

4. Composition pharmaceutique selon la revendication 3, dans laquelle la quantité du principe actif pharmaceutique sensible à l'humidité est d'environ 0,6% à environ 2,7% par rapport au poids total de la composition.

5. Composition pharmaceutique selon l'une quelconque des revendications précédentes, dans laquelle le principe actif pharmaceutique sensible à l'humidité est le cilazapril.

6. Composition pharmaceutique selon l'une quelconque des revendications précédentes, dans laquelle la quantité du deuxième principe actif pharmaceutique sensible à l'humidité est d'environ 1% à environ 25% par rapport au poids total de la composition.

7. Composition pharmaceutique selon la revendication 6, dans laquelle la quantité du deuxième principe actif pharmaceutique est d'environ 6% à environ 10% par rapport au poids total de la composition.

8. Composition pharmaceutique selon l'une quelconque des revendications précédentes, dans laquelle le deuxième principe actif pharmaceutique est l'hydrochlorothiazide.

9. Composition pharmaceutique selon l'une quelconque des revendications précédentes, dans laquelle au moins un excipient pharmaceutique est un liant.

10. Composition pharmaceutique selon la revendication 9, dans laquelle le liant est choisi dans le groupe constitué par des dérivés de la cellulose, les polyvinylpyrrolidones et leurs dérivés, les poly(acétate de vinyle) et les poly(alcool de vinyle).

11. Composition pharmaceutique selon la revendication 10, où le liant est choisi dans le groupe constitué par la copovidone et l'hypromellose.

12. Composition pharmaceutique selon l'une quelconque des revendications 9 à 11, dans laquelle la quantité du liant est d'au moins environ 4% par rapport au poids total de la composition.

13. Composition pharmaceutique selon la revendication 12, dans laquelle la quantité du liant est d'environ 4% à environ 20% par rapport au poids total de la composition.

14. Composition pharmaceutique selon la revendication 13, dans laquelle la quantité du liant est d'environ 5% à environ 10% par rapport au poids total de la composition.

15. Composition pharmaceutique selon l'une quelconque des revendications précédentes, dans laquelle le principe actif pharmaceutique sensible à l'humidité présente un produit de dégradation principal et dans laquelle la composition comprend ce produit de dégradation principal en une quantité ne dépassant pas environ 3% en poids par rapport au poids initial total du principe actif pharmaceutique sensible à l'humidité dans la composition pharmaceutique après stockage.

16. Composition pharmaceutique selon la revendication 15, où la quantité du produit de dégradation principal du principe actif pharmaceutique sensible à l'humidité dans la composition pharmaceutique ne dépasse pas environ 2% en poids par rapport au poids initial total du principe actif pharmaceutique sensible à l'humidité.

17. Composition pharmaceutique selon la revendication 16, où la quantité du produit de dégradation principal du principe actif pharmaceutique sensible à l'humidité dans la composition pharmaceutique ne dépasse pas environ 1% en poids par rapport au poids initial total du principe actif pharmaceutique sensible à l'humidité.

18. Composition pharmaceutique selon l'une quelconque des revendications 15 à 17, dans laquelle le stockage est dans un emballage présentant des propriétés de barrière à l'humidité, qui sont au moins aussi efficaces que celles de coques aluminium-aluminium formées à froid.

19. Composition pharmaceutique selon la revendication 18, dans laquelle le stockage se fait à 55°C pendant quatre semaines.

20. Composition pharmaceutique selon l'une quelconque des revendications précédentes, dans laquelle la composition est sous une forme de dosage solide.

21. Composition pharmaceutique selon la revendication 20, dans laquelle la forme de dosage est choisie parmi le groupe constitué par un comprimé et une gélule.

22. Composition pharmaceutique selon la revendication 21, dans laquelle la forme de dosage est un comprimé.

23. Composition pharmaceutique selon la revendication 22, dans laquelle le comprimé comprend un enrobage esthétique pour comprimés.

24. Composition pharmaceutique selon la revendication 23, dans laquelle l'enrobage esthétique pour comprimés présente des propriétés de barrière à l'humidité.

25. Composition pharmaceutique selon la revendication 24, dans laquelle l'enrobage esthétique pour comprimés présentant des propriétés de barrière à l'humidité est choisi dans le groupe constitué par des enrobages pour comprimés des séries Opadry® 85F.

26. Composition pharmaceutique selon l'une quelconque des revendications 23 à 25, dans laquelle l'enrobage esthétique pour comprimés représente environ 2% à environ 6% en poids par rapport au poids du comprimé.

27. Composition pharmaceutique selon la revendication 26, dans laquelle la quantité de l'enrobage esthétique pour comprimés est d'environ 3% à environ 3,5% par rapport au poids du comprimé.

28. Procédé de préparation d'une composition pharmaceutique comprenant un principe actif pharmaceutique sensible à l'humidité et un deuxième principe actif pharmaceutique comprenant les étapes suivantes consistant à :
a) fournir un principe actif pharmaceutique sensible à l'humidité ;
b) mélanger le principe actif pharmaceutique sensible à l'humidité avec au moins un excipient acceptable sur le plan pharmaceutique, formant un mélange ; et
c) granuler par voie humide le mélange avec une solution d'un excipient du liant dissous dans un ou plusieurs solvants de traitement formant un granulé humide ;
d) fournir une matière comprenant un deuxième principe actif pharmaceutique et éventuellement un ou plusieurs excipients pharmaceutiques ; et
e) ajouter la matière provenant de l'étape d) au granulé humide de l'étape c) formant un granulé combiné,
dans laquelle lorsque la matière de l'étape d) comprend un deuxième principe actif pharmaceutique et un ou plusieurs excipients pharmaceutiques, la matière est éventuellement un mélange obtenu par le mélange du deuxième principe actif pharmaceutique avec un ou plusieurs excipients pharmaceutiques.

29. Procédé selon la revendication 28, dans lequel la quantité du principe actif pharmaceutique sensible à l'humidité est d'environ 0,1% à environ 25% et la quantité du deuxième principe actif pharmaceutique est d'environ 1% à environ 25% par rapport au poids total de la composition.

30. Procédé selon la revendication 29, dans lequel la quantité du principe actif pharmaceutique sensible à l'humidité est d'environ 0,6% à environ 2,7% et la quantité du deuxième principe actif pharmaceutique est d'environ 5% à environ 10% par rapport au poids total de la composition.

31. Procédé selon l'une quelconque des revendications 28 à 30, dans lequel le principe actif pharmaceutique sensible à l'humidité est le cilazapril et le deuxième principe actif pharmaceutique est l'hydrochlorothiazide.

32. Procédé selon l'une quelconque des revendications 28 à 31, dans lequel le liant est choisi dans le groupe constitué par des dérivés de la cellulose, les polyvinylpyrrolidones et leurs dérivés, les poly(acétate de vinyle) et les poly(alcool de vinyle).

33. Procédé selon la revendication 32, dans lequel le liant est choisi dans le groupe constitué par la copovidone et l'hypromellose.

34. Procédé selon l'une quelconque des revendications 28 à 33, dans lequel la quantité du liant est d'au moins environ 4% en poids par rapport au poids total de la composition.

35. Procédé selon la revendication 34, dans lequel la quantité du liant est d'environ 5% à environ 10% par rapport au poids total de la composition.

36. Procédé selon l'une quelconque des revendications 28 à 35, dans lequel le solvant de traitement est choisi dans le groupe constitué par l'éthanol, l'isopropanol, l'eau et leurs combinaisons.

37. Procédé selon l'une quelconque des revendications 28 à 36, dans lequel le liant est appliqué en tant que solution dans l'eau or l'éthanol.

38. Procédé selon la revendication 37, dans lequel la solution du liant dans l'eau ou l'éthanol comprend environ 25% à environ 55% (p/p) du liant.

39. Procédé selon la revendication 38, dans lequel la solution du liant dans l'eau ou l'éthanol comprend environ 30% à environ 50% (p/p) du liant.

40. Procédé selon l'une quelconque des revendications 28 à 39 de préparation d'une composition pharmaceutique, dans lequel le procédé comprend en outre les étapes consistant à :
f) mélanger le granulé combiné provenant de l'étape e) avec un ou plusieurs excipients formant un mélange final ;
g) presser le mélange final en un comprimé ; et
h) enrober éventuellement le comprimé avec un enrobage esthétique.

41. Procédé selon la revendication 40, dans lequel l'étape d'enrobage du comprimé consiste à préparer une suspension comprenant environ 10% à environ 15% d'un mélange de poudre pour enrobage esthétique et à appliquer la suspension sur le comprimé.

42. Procédé selon la revendication 41, dans lequel la suspension comprend environ 12% à environ 13% d'un mélange de poudre pour enrobage esthétique.

43. Procédé selon la revendication 41 ou 42, dans lequel l'enrobage esthétique présente des propriétés de barrière à l'humidité et le mélange de poudre pour enrobage esthétique est choisi parmi les mélanges de poudres des séries Opadry® 85F.

44. Procédé selon l'une quelconque des revendications 28 à 39 de préparation d'une composition pharmaceutique, dans lequel le procédé comprend en outre les étapes consistant à mélanger le granulé avec un ou plusieurs excipients formant un mélange final et remplir une gélule avec le mélange final.

45. Procédé selon l'une quelconque des revendications 28 à 44, dans lequel le ou les excipients acceptables sur le plan pharmaceutique utilisés dans l'étape b) ne comprennent pas de liant.

46. Composition pharmaceutique pouvant être obtenue par un procédé tel que défini dans l'une quelconque des revendications 28 à 45.

47. Composition pharmaceutique telle que définie dans l'une quelconque des revendications 1 à 27 pour une utilisation en thérapie.

48. Composition pharmaceutique telle que définie dans l'une quelconque des revendications 1 à 27 pour traiter l'hypertension.

49. Utilisation du cilazapril dans la préparation d'une composition pharmaceutique telle que définie dans l'une quelconque des revendications 1 à 27 pour traiter l'hypertension.
